# EUROPEAN PATENT APPLICATION

(11) **EP 3 881 847 A1**
(43) Date of publication of application: **22.09.2021**
(21) Application number: 19884180.1
(22) Date of filing: 14.11.2019
(51) Int. Cl.: A61K 31/506, A61K 45/00, A61P 35/00

(54) **PHARMACEUTICAL COMPOSITION FOR TREATING DIFFUSE-TYPE GASTRIC CANCER**

(30) Priority: 14.11.2018 JP 2018213496
(71) Applicant: Kanazawa Medical University, Uchinada-machi Kahoku-gun Ishikawa 920-0293 (JP)
(72) Inventor: YASUMOTO Kazuo, Kahoku-gun, Ishikawa 920-0293 (JP)
(74) Representative: Michalski Hüttermann & Partner Patentanwälte mbB
(86) International application number: PCT/JP2019/044662
(87) International publication number: WO 2020/100969

(57) **Abstract**

An object of the present invention is to provide a molecular targeted drug effective for diffuse gastric cancer. According to the present invention, there is provided a pharmaceutical composition for treating diffuse gastric cancer in a patient, which comprises an EGF receptor inhibitor having an ErbB1 inhibitory activity and an ErbB4 inhibitory activity. According to the present invention, there is provided the aforementioned pharmaceutical composition for use in a combination therapy with an anti-VEGF receptor 2 antibody and/or a cMET inhibitor. According to the present invention, there is provided a method for treating diffuse gastric cancer in a patient, which comprises the step of administering an EGF receptor inhibitor having an ErbB1 inhibitory activity and an ErbB4 inhibitory activity to the patient.

## Description

### Technical field

The present invention relates to a pharmaceutical composition for treating diffuse gastric cancer comprising an EGF receptor inhibitor. The present invention relates to a pharmaceutical composition for treating diffuse gastric cancer comprising an EGF receptor inhibitor for use in combination therapy with an anti-VEGF receptor 2 antibody and/or a cMET inhibitor.

### Cross-reference of Related Application

This application claims convention priority based on Japanese Patent Application No. 2018-213496, filed on November 14, 2018, of which entire disclosure is incorporated herein by reference.

### Background Art

Diffuse gastric cancer consists of poorly differentiated adenocarcinoma, signet ring cell carcinoma, and so forth, and tends to progress faster and provides worse prognosis compared with non-diffuse gastric cancer consisting of highly differentiated adenocarcinoma. In particular, scirrhous gastric cancer, which is included in diffuse gastric cancer, is characterized by having a high degree of stromal fibrosis, and diffuse infiltration of cancer cells, and often having undifferentiated adenocarcinoma cells (Non-patent document 1). Scirrhous gastric cancer highly frequently develops peritoneal carcinomatosis, which consists of peritoneal dissemination of cancer cells and accumulation of large volume of ascites, and thereby provides very poor prognosis (Non-patent documents 1 and 2).

The SP therapy using S-1 (tegafur, gimeracil and potassium oteracil-containing agent) and cisplatin (CDDP) is performed as a standard treatment for unresectable advanced or recurrent gastric cancer (Non-patent document 3). On the other hand, with the advent of the anti-HER2 therapy, personalized medicine mainly based on anti-HER2 therapeutic agents attracts attention also in the gastric cancer treatment. Although survival-improving effect of trastuzumab has been revealed for HER2-positive gastric cancer in the international corporative study (ToGA), the ratio of HER2-positive gastric cancers to the total gastric cancers is not high (about 10 to 20%). Therefore, also for gastric cancers other than HER2-positive gastric cancer, more useful novel molecular targeted drugs that can contribute to personalized medicine are being searched for.

Gastric cancers are classified into "early stage gastric cancers" and "advanced gastric cancers" according to the depth of invasion, and it is said that diffuse gastric cancers account for 50 to 60% of advanced gastric cancers, in which cancer invades into tissues deeper than the intrinsic muscular layer, and scirrhous gastric cancer accounts for around 20% of them. However, any effective molecular targeted drug for advanced diffuse gastric cancer is not known. For example, an international cooperative study was conducted for verifying add-on efficacy of bevacizumab (AVAGAST) in chemotherapy for advanced recurrent gastric cancers, but such efficacy was not confirmed. In addition, the study for verifying add-on efficacy of panitumumab or cetuximab in chemotherapy for advanced gastric cancer also failed to confirm efficacy thereof (REAL3 and EXPAND trials).

### Prior art references

### Non-patent documents

Non-patent document 1: Zhao L. et al., Cancer Science, December 2013, vol. 104, No. 12, pp.1640-1646
Non-patent document 2: Yasumoto K. et al., Clin. Cancer Res., 2011;17:3619-3630
Non-patent document 3: Koizumi W. et al., Lancet Oncol., 9(3):215-21(2008)
Non-patent document 4: Zhang et al., Journal of Hematology & Oncology, 2014, 7:22
Non-patent document 5: Hua Xie et al., 011, PLoS ONE, 6(7): e21487, doi:10.1371/journal.pone.0021487
Non-patent document 6: James W.T. Yates et al., 2016, Mol. Cancer Ther.; 15(10);2378-87
Non-patent document 7: Nicolas Floc'h et al., 2018, Mol. Cancer Ther.; 17(5); 885-96
Non-patent document 8: Cross D.A.E. et al., Cancer Discov., 2014 Sep; 4(9): 1046-1061
Non-patent document 9: Modjtahedi H., et al., 2014, Naunyn-Schmiedeberg's Arch. Pharmacol., 387:505-52
Non-patent document 10: Cha MY et al., 2012, Int. J. Cancer, 130, 2445-2454
Non-patent document 11: Engelman J.A. et al., Cancer Res., 2007;67(24):11924-32
Non-patent document 12: Traxler p. et al., CANCER RESEARCH, 64, 4931-4941, July 15, 2004
Non-patent document 13: Wong T. W. et al., Clin. Cancer Res., 2006;12(20), October 15, 2006
Non-patent document 14: Doi T. et al., British Journal of Cancer (2012) 106, 666-672
Non-patent document 15: Amemiya et al., Oncology 2002; 63: 286-96, Park et al, APMIS 2000; 108: 195-200
Non-patent document 16: Olayioye MA et al., EMBO J. 2000; 19:3159-67
Non-patent document 17: Nakashio T et al., Int. J. Cancer, 1997;70:612-8
Non-patent document 18: Nakajima M et al., Cancer, 1999;85:1894-1902
Non-patent document 19: Kalluri R et al., Nat. Rev. Cancer, 2006;6:392-401

The entire descriptions of Non-patent documents 1 to 19 are incorporated herein by reference.

### Summary of the Invention

### Object to be Achieved by the Invention

Diffuse gastric cancers including scirrhous gastric cancer and associated with carcinomatous peritonitis account for about half of all recurrent and advanced gastric cancers, but the problem is that there is not known any effective molecular targeted drug for this type of cancer. There is also a problem that no effective therapy is currently available for scirrhous gastric cancer, which provides an extremely poor prognosis due to development of carcinomatous peritonitis in a short period of time.

Although individualized therapies for HER2-positive gastric cancers have been provided so far, any effective molecular targeted drug is not known for diffuse gastric cancer that is not HER2-positive gastric cancer. Therefore, it is a challenge to provide a molecular targeted drug effective for diffuse gastric cancer. An object of the present invention is to provide a novel treatment method for diffuse gastric cancer and a pharmaceutical composition used for the treatment.

### Means for Achieving the Object

As a result of the intensive researches of the inventor of the present invention to achieve the aforementioned object, it was found that specific EGF receptor inhibitors have an anticancer action on diffuse gastric cancer cells. The present invention is based on this finding.

The present invention provides the followings.
[1] A pharmaceutical composition for treating diffuse gastric cancer in a patient, which comprises an EGF receptor inhibitor having an ErbB1 inhibitory activity and an ErbB4 inhibitory activity.
[2] The pharmaceutical composition according to [1], which is for use in a combination therapy with an anti-VEGF receptor 2 antibody and/or a cMET inhibitor.
[3] The pharmaceutical composition according to [2], wherein the EGF receptor inhibitor is simultaneously or sequentially administered with the anti-VEGF receptor 2 antibody and/or the cMET inhibitor in the combination therapy.
[4] The pharmaceutical composition according to any one of [1] to [3], wherein the EGF receptor inhibitor is any one selected from the group consisting of afatinib (BIBW2992), AZ5104, osimertinib (AZD9291), poziotinib (HM781-36B), dacomitinib (PF299804, PF299), AEE788 (NVP-AEE788), AC480 (BMS-599626), TAK-285, lapatinib (GW-572016) ditosylate, and lapatinib, or a pharmaceutically acceptable salt thereof.
[5] The pharmaceutical composition according to any one of [1] to [4], wherein the EGF receptor inhibitor is any one selected from the group consisting of AZ5104 and osimertinib (AZD9291), or a pharmaceutically acceptable salt thereof.
[6] The pharmaceutical composition according to any one of [2] to [5], wherein the anti-VEGF receptor 2 antibody is ramucirumab.
[7] The pharmaceutical composition according to any one of [2] to [6], wherein the cMET inhibitor is savolitinib.
[8] The pharmaceutical composition according to any one of [1] to [7], wherein the diffuse gastric cancer is scirrhous gastric cancer.
[9] The pharmaceutical composition according to any one of [1] to [8], wherein the patient suffers from carcinomatous peritonitis.
[10] A method for treating diffuse gastric cancer in a patient, which comprises the step of administering an EGF receptor inhibitor having an ErbB1 inhibitory activity and an ErbB4 inhibitory activity to the patient.
[11] The treatment method according to [10], wherein the administration step comprises administering the EGF receptor inhibitor simultaneously or sequentially with an anti-VEGF receptor 2 antibody.
[12] The treatment method according to [11], wherein the administration step comprises administering the EGF receptor inhibitor and the anti-VEGF receptor 2 antibody simultaneously or sequentially with a cMET inhibitor.
[13] The treatment method according to any one of [10] to [12], wherein the EGF receptor inhibitor is any one selected from the group consisting of afatinib (BIBW2992), AZ5104, osimertinib (AZD9291), poziotinib (HM781-36B), dacomitinib (PF299804, PF299), AEE788 (NVP -AEE788), AC480 (BMS-599626), TAK-285, lapatinib (GW-572016) ditosylate, and lapatinib, or a pharmaceutically acceptable salt thereof.
[14] The treatment method according to in any one of [10] to [13], wherein the EGF receptor inhibitor is any one selected from the group consisting of AZ5104 and osimertinib (AZD9291), or a pharmaceutically acceptable salt thereof.
[15] The treatment method according to any one of [11] to [14], wherein the anti-VEGF receptor 2 antibody is ramucirumab.
[16] The treatment method according to any one of [12] to [15], wherein the cMET inhibitor is savolitinib.
[17] The treatment method according to any one of [10] to [16], wherein the diffuse gastric cancer is scirrhous gastric cancer.
[18] The treatment method according to any one of [10] to [17], wherein the patient suffers from carcinomatous peritonitis.
[19] A pharmaceutical composition for treating diffuse gastric cancer in a patient, which comprises an EGF receptor inhibitor having an ErbB1 inhibitory activity and an ErbB4 inhibitory activity, and is for use in a combination therapy with an anti-VEGF receptor 2 antibody.
[20] A pharmaceutical composition for treating diffuse gastric cancer in a patient, which comprises an EGF receptor inhibitor having an ErbB1 inhibitory activity and an ErbB4 inhibitory activity, and is for use in a combination therapy with a cMET inhibitor.
[21] A pharmaceutical composition for treating diffuse gastric cancer in a patient, which comprises an EGF receptor inhibitor having an ErbB1 inhibitory activity and an ErbB4 inhibitory activity, and is for use in a combination therapy with an anti-VEGF receptor 2 antibody and a cMET inhibitor.
[22] A pharmaceutical composition for treating diffuse gastric cancer in a patient, which comprises an anti-VEGF receptor 2 antibody, and is for use in a combination therapy with an EGF receptor inhibitor having an ErbB1 inhibitory activity and an ErbB4 inhibitory activity.
[23] A pharmaceutical composition for treating diffuse gastric cancer in a patient, which comprises an anti-VEGF receptor 2 antibody, and is for use in a combination therapy with an EGF receptor inhibitor having an ErbB1 inhibitory activity and an ErbB4 inhibitory activity and a cMET inhibitor.
[24] A pharmaceutical composition for treating diffuse gastric cancer in a patient, which comprises a cMET inhibitor, and is for use in a combination therapy with an EGF receptor inhibitor having an ErbB1 inhibitory activity and an ErbB4 inhibitory activity.
[25] A pharmaceutical composition for treating diffuse gastric cancer in a patient, which comprises a cMET inhibitor, and is for use in a combination therapy with an EGF receptor inhibitor having an ErbB1 inhibitory activity and an ErbB4 inhibitory activity and an anti-VEGF receptor 2 antibody.
[26] The pharmaceutical composition according to any one of [19] to [25], wherein the EGF receptor inhibitor is administered simultaneously or sequentially with the anti-VEGF receptor 2 antibody and/or the cMET inhibitor in the combination therapy.
[27] The pharmaceutical composition according to any one of [19] to [26], wherein the EGF receptor inhibitor is selected from the group consisting of afatinib (BIBW2992), AZ5104, osimertinib (AZD9291), poziotinib (HM781-36B), dacomitinib (PF299804, PF299), AEE788 (NVP -AEE788), AC480 (BMS-599626), TAK-285, lapatinib (GW-572016) ditosylate, and lapatinib, or a pharmaceutically acceptable salt thereof.
[28] The pharmaceutical composition according to any one of [19] to [27], wherein the EGF receptor inhibitor is any one selected from the group consisting of AZ5104 and osimertinib (AZD9291), or a pharmaceutically acceptable salt thereof.
[29] The pharmaceutical composition according to any one of [19] to [28], wherein the anti-VEGF receptor 2 antibody is ramucirumab.
[30] The pharmaceutical composition according to any one of [19] to [29], wherein the cMET inhibitor is savolitinib.
[31] The pharmaceutical composition according to any one of [19] to [30], wherein the diffuse gastric cancer is scirrhous gastric cancer.
[32] The pharmaceutical composition according to any one of [19] to [31], wherein the patient suffers from carcinomatous peritonitis.

### Brief Description of the Drawings

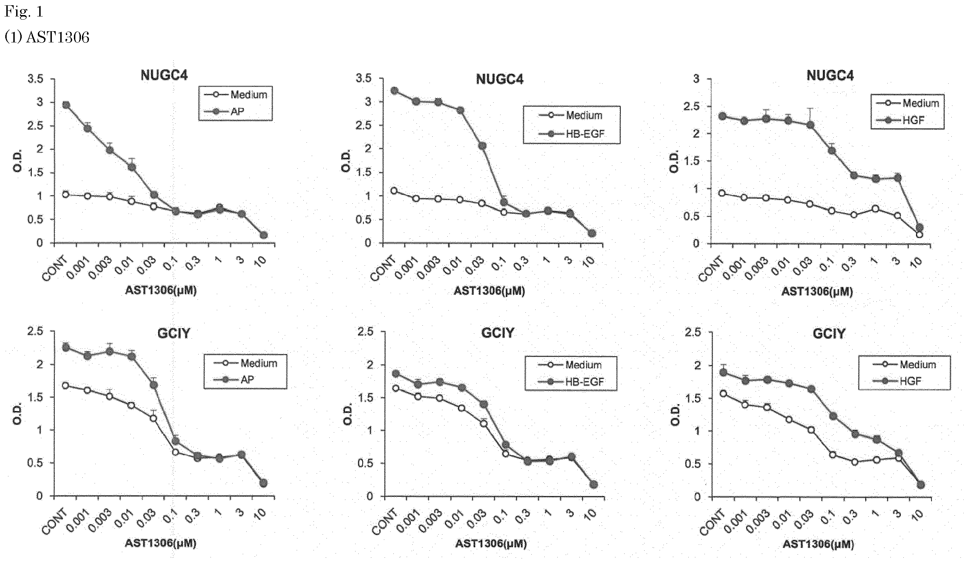

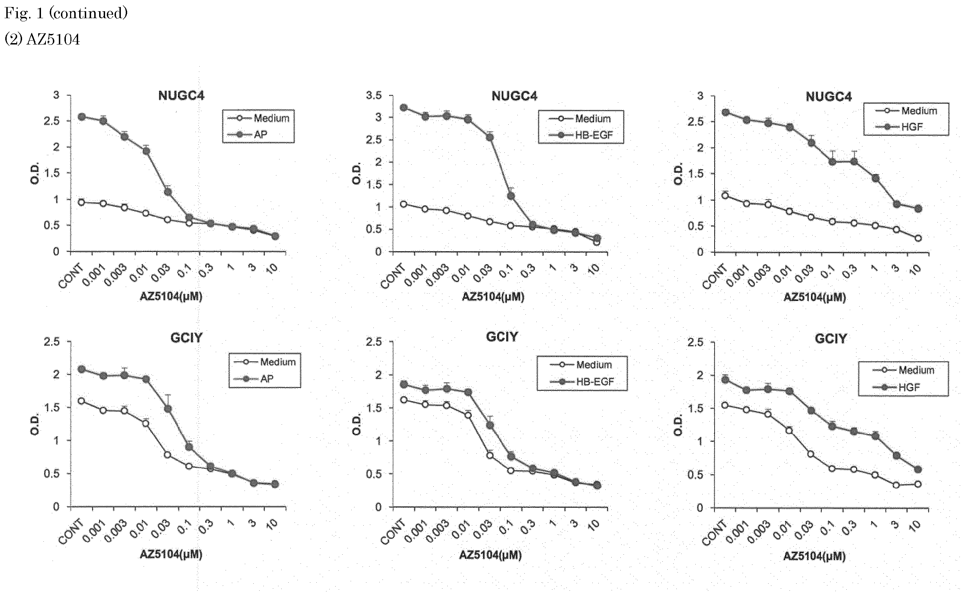

[Fig. 1] Fig. 1 shows results of cell growth inhibition studies performed with EGF receptor inhibitors, (1) AST1306 and (2) AZ5104, for the diffuse gastric cancer cell lines, NUGC4 and GCIY. The abbreviation "AP" indicates a gastric cancer cell line cultured in the presence of amphiregulin and the EGF receptor inhibitor. The abbreviation "HB-EGF" indicates a gastric cancer cell line cultured in the presence of HB-EGF and the EGF receptor inhibitor. The abbreviation "HGF" indicates a gastric cancer cell line cultured in the presence of HGF and the EGF receptor inhibitor. "Medium" indicates a gastric cancer cell line cultured in the presence of the EGF receptor inhibitor without adding any of AP, HB-EGF, and HGF.

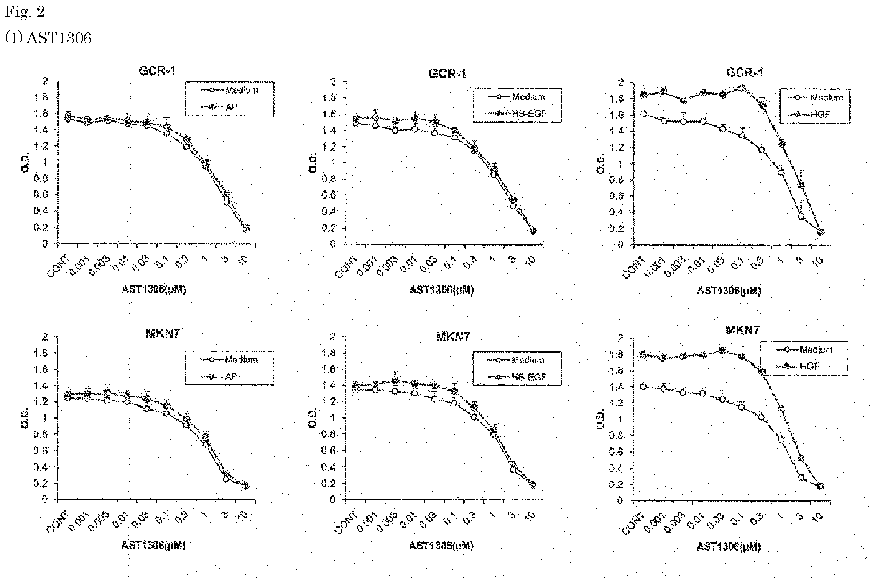

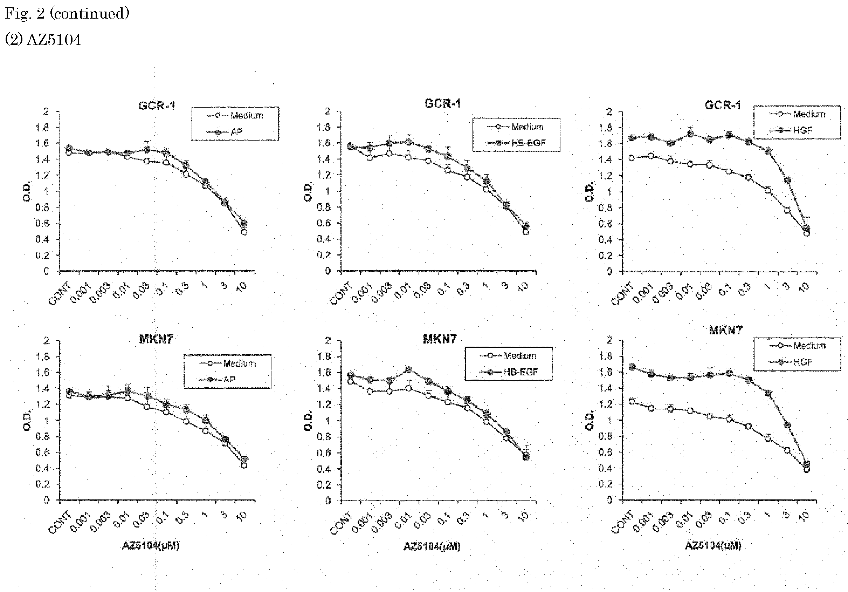

[Fig. 2] Fig. 2 shows results of cell growth inhibition studies performed with EGF receptor inhibitors, (1) AST1306 and (2) AZ5104, for the non-diffuse gastric cancer cell lines, GCR-1 and MKN7. The abbreviation "AP" indicates a gastric cancer cell line cultured in the presence of amphiregulin and the EGF receptor inhibitor. The abbreviation "HB-EGF" indicates a gastric cancer cell line cultured in the presence of HB-EGF and the EGF receptor inhibitor. The abbreviation "HGF" indicates a gastric cancer cell line cultured in the presence of HGF and the EGF receptor inhibitor. "Medium" indicates a gastric cancer cell line cultured in the presence of the EGF receptor inhibitor without adding any of AP, HB-EGF, and HGF.

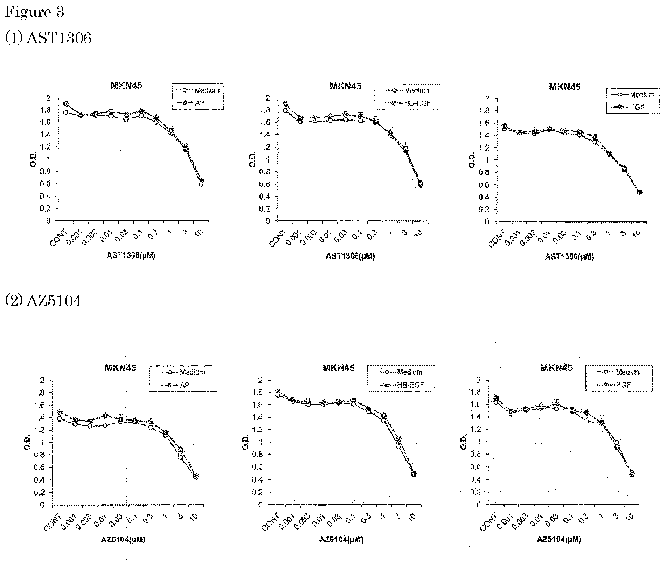

[Fig. 3] Fig. 3 shows results of cell growth inhibition studies performed with the EGF receptor inhibitors, (1) AST1306 and (2) AZ5104, for the cMet gene-amplified gastric cancer cell line MKN45. The abbreviation "AP" indicates a gastric cancer cell line cultured in the presence of amphiregulin and the EGF receptor inhibitor. The abbreviation "HB-EGF" indicates a gastric cancer cell line cultured in the presence of HB-EGF and the EGF receptor inhibitor. The abbreviation "HGF" indicates a gastric cancer cell line cultured in the presence of HGF and the EGF receptor inhibitor. "Medium" indicates a gastric cancer cell line cultured in the presence of the EGF receptor inhibitor without adding any of AP, HB-EGF, and HGF.

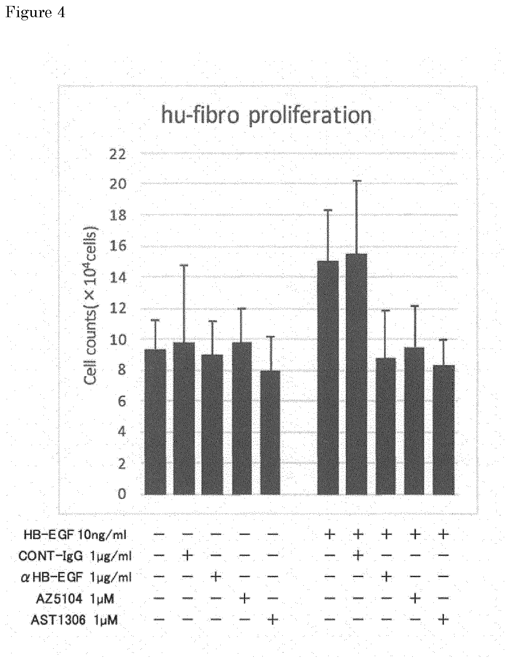

[Fig. 4] Fig. 4 shows results of in vitro growth inhibition studies performed with the EGF receptor inhibitors, AST1306 and AZ5104, for normal human gastric fibroblasts. In the line of "HB-EGF 10 ng/ml", it is indicated that the culture of fibroblasts was performed in the presence (+) or absence (-) of such an amount of human HB-EGF. In the line of "CONT-IgG 1 µg/ml", it is indicated that the culture of fibroblasts was performed in the presence (+) or absence (-) of such an amount of human normal IgG. In the line of "αHB-EGF 1 µg/ml", it is indicated that the culture of fibroblasts was performed in the presence (+) or absence (-) of such an amount of anti-human HB-EGF antibody. In the line of "AZ5104 1 µM", it is indicated that the culture of fibroblasts was performed in the presence (+) or absence (-) of such an amount of AZ5104. In the line of "AST1306 1 µM", it is indicated that the culture of fibroblasts was performed in the presence (+) or absence (-) of such an amount of AST1306.

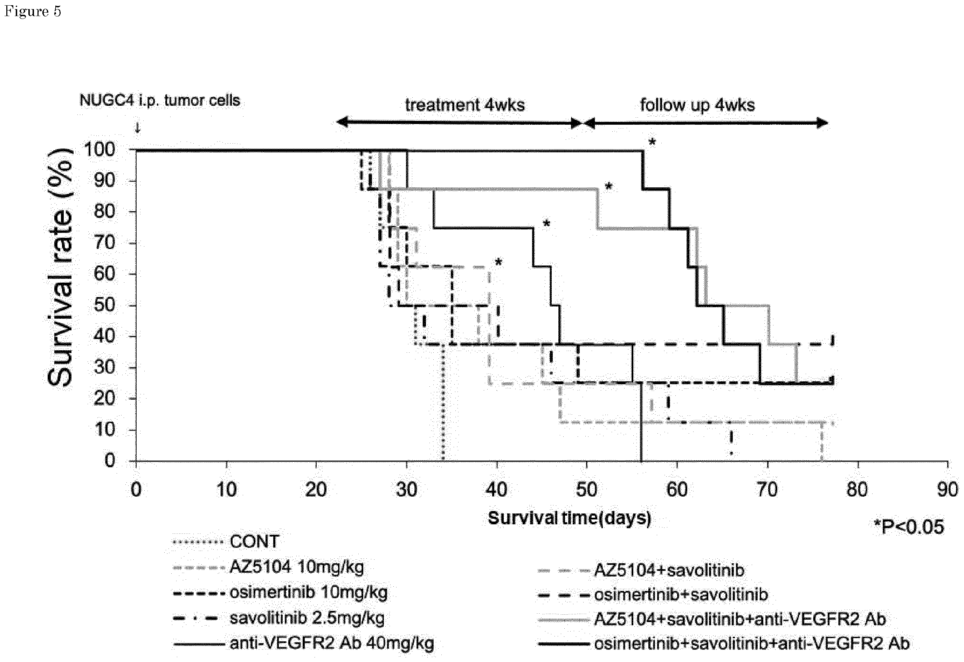

[Fig. 5] Fig. 5 shows survival curves for the eight groups and the control group tested in vivo in Example 3. The vertical axis indicates the survival rate, and the horizontal axis indicates the number of days. The survival curves were created by the Kaplan-Meier method, and the log-rank test was performed. *P<0.05 is for comparison with the control group.

### Modes for Carrying out the Invention

The following explanations of the present invention may be made by referring to representative embodiments or specific examples, but the present invention is not limited to such embodiments or specific examples. In this specification, a numerical range expressed with "to" means a range including the numerical values described before and after "to" as the lower and upper limits.

### (Pharmaceutical composition)

The present invention provides a pharmaceutical composition for treating diffuse gastric cancer in a patient, which comprises an EGF receptor inhibitor having an ErbB1 inhibitory activity and an ErbB4 inhibitory activity. The patient may be a mammal including a human.

Gastric cancers are classified into types 1 to 4 according to the classification method based on the morphologies thereof observed with naked eyes (Borrmann classification). Those that cannot be classified into the types 1 to 4 may be classified into type 5. In this specification, the term "diffuse gastric cancer" refers to those of the type 3 (infiltrative ulcerative type) and type 4 (diffuse infiltrative type), while "scirrhous gastric cancer" refers to those of the type 4 (diffuse infiltrative type). In this specification, the term "diffuse gastric cancer" includes scirrhous gastric cancer, unless especially specified.

EGF receptor inhibitors (herein also referred to as EGFR inhibitors) generally reduce or prevent the expression and/or activity of the EGF receptors. Appropriate EGF receptor inhibitors can be identified by performing assay for inhibitory effects on the EGF receptor expression and/or activity. The inhibitors reduce the expression or activity by at least a degree detectable in an appropriate assay. The inhibitors can reduce the expression or activity by, for example, at least 10, 20, 30, 40, 50, 60, 70, or 80%, or at least 90%. An arbitrary appropriate EGF receptor inhibitor may have a tyrosine kinase inhibitory activity and/or inhibit signaling from the EGF receptor.

The EGF receptors are tyrosine kinase type receptors, and belong to especially a type of receptors known as the ErbB family. The ErbB family includes 4 types, ErbB1 (EGFR), ErbB2 (HER2), ErbB3 (HER3), and ErbB4 (HER4).

As the aforementioned EGF receptor inhibitor, one that has an ErbB1 inhibitory activity and an ErbB4 inhibitory activity can be selected. The aforementioned EGF receptor inhibitor may have an ErbB2 inhibitory activity in addition to the ErbB1 and ErbB4 inhibitory activities, or may not have any ErbB2 inhibitory activity.

Examples of the aforementioned EGF receptor inhibitors include AST1306, afatinib (BIBW2992), AZ5104, osimertinib (AZD9291), poziotinib (HM781-36B), dacomitinib (PF299804, PF299), AEE788 (NVP-AEE788), AC480 (BMS-599626), TAK-285, lapatinib (GW-572016) ditosylate, lapatinib, and so forth. The inhibitors can include pharmaceutically acceptable salts of those mentioned above, which will be described below.

AST1306 (allitinib) is 2-propenamide, N-[4-[[3-chloro-4-[(3-fluorophenyl)methoxy]phenyl]amino]-6-quinazolinyl]-, 4-methylbenzenesulfonate (1:1) (CAS Registry Number: 1050500-29-2). AZ5104 is N-(5-(4-(1H-indol-3-yl)pyrimidin-2-ylamino)-2-((2-(dimethylamino)ethyl)(methyl)amino)-4-methoxyphenyl)acrylamide (CAS Registry Number: 1421373-98-9). Osimertinib (AZD9291) is 2-propenamide, N-[2-[[2-(dimethylamino)ethyl]methylamino]-4-methoxy-5-[[4-(1-methyl-1H-indol-3-yl)-2-pyrimidinyl]amino]phenyl]-(CAS Registry Number: 1421373-65-0). Poziotinib (HM781-36B) is 1-(4-(4-(3,4-dichloro-2-fluorophenylamino)-7-methoxyquinazolin-6-yloxy)piperidin-1-yl)prop-2-en-1-one (CAS Registry Number: 1092364-38-9). AEE788 (NVP-AEE788) is (R)-6-(4-((4-ethylpiperazin-1-yl)methyl)phenyl)-N-(1-phenylethyl)-7H-pyrrolo[2,3-d]pyrimidin-4-amine (CAS Registry Number: 497839-62-0). AC480 (BMS-599626) is (S)-morpholin-3-ylmethyl 4-(1-(3-fluorobenzyl)-1H-indazol-5-ylamino)-5-methylpyrrolo[1,2-f][1,2,4]triazine-6-carbamate (CAS Registry Number: 714971-09-2). TAK-285 is N-(2-(4-(3-chloro-4-(3-(trifluoromethyl)phenoxy)phenylamino)-5H-pyrrolo[3,2-d]pyrimidin-5-yl)ethyl)-3-hydroxy-3-methylbutanamide (CAS Registry Number: 871026-44-7).

According to one embodiment, the EGF receptor inhibitor is AST1306, AZ5104, or osimertinib (AZD9291). The EGF receptor inhibitors are available from Selleck Chemicals, Santa Cruz Biotechnology, and so forth.

The pharmaceutically acceptable salt may be, for example, but are not limited to, an acid addition salt that is sufficiently basic, e.g., an acid addition salts with an inorganic or organic acid. Examples of the acid addition salt include fumarate, stearate, benzoate, acetate, succinate, tartrate, lactate, methanesulfonate, ethanesulfonate, p-toluenesulfonate, benzenesulfonate, hydrochloride, hydrobromide, citrate and maleate, as well as salts formed with phosphorus or sulfuric acid, but are not limited to these.

The pharmaceutically acceptable salt may be, but are not limited to, a salt that is sufficiently acidic, e.g., an alkali or alkaline earth metal salt. Examples of the alkali or alkaline earth metal salt include salts with an alkali metal (e.g., sodium or potassium), salts with an alkaline earth metal (e.g., calcium or magnesium), ammonium salts, salts with an organic amine (e.g., triethylamine, ethanolamine, diethanolamine, triethanolamine, morpholine, N-methylpiperidine, N-ethylpiperidine, and dibenzylamine), and salts with an amino acid (e.g., lysine), but are not limited to these. The salts may be, for example, a mesylate or tosylate.

AST1306, exemplified as the EGF receptor inhibitor, is an irreversible inhibitor of EGFR (ErbB1), HER2 (ErbB2) and HER4 (ErbB4) signaling (Zhang et al., Journal of Hematology & Oncology, 2014, 7:22). Hua Xie et al. (011, PLoS ONE 6(7): e21487. doi:10.1371/journal.pone.0021487) reported that AST1306 is an anilino-quinazoline compound, and is a selective and irreversible ErbB2 and EGFR inhibitor, the growth inhibitory effect thereof is more potent in ErbB2-overexpressing cells, and in vivo, AST1306 potently inhibited tumor growth in ErbB2-overexpressing adenocarcinoma xenografted mice and FVB-2/Nneu transgenic breast cancer mice, but weakly inhibited tumor growth in EGFR-overexpressing tumor xenografted mice. Further, a phase I open-label dose-escalation study using AST1306 resulted in partial remission in 7 patients and stability (stability over 6 months or longer) in 7 patients among 55 cancer patients who participated in the study and could be evaluated (Zhang et al., Journal of Hematology & Oncology, 2014, 7:22).

Osimertinib (AZD9291), exemplified as the EGF receptor inhibitor, is approved in the United States, Europe, Japan, and other countries to be sold as a therapeutic agent for advanced non-small cell lung cancer with EGFR T790M mutation. AZ5104, exemplified as the EGF receptor inhibitor, is an active metabolite of osimertinib (AZD9291) (James W.T. Yates et al., 2016, Mol. Cancer Ther., 15(10);2378-87). It has also been reported that osimertinib (AZD 9291) and AZ5104 inhibit tumor growth in mice transplanted with non-small cell lung cancer with EGFR EX20Ins mutation (Nicolas Floc'h et al., 2018, Mol. Cancer Ther., 17(5);885 -96). Osimertinib (AZD9291) and AZ5104 have been reported to have inhibitory activities also against wild-type EGFR, ErbB2, and ErbB4, in addition to the inhibitory activity against mutant EGFR (Cross D.A.E. et al., Cancer Discov., 2014 Sep; 4(9): 1046-1061.)

It has been reported that all of afatinib (BIBW2992), poziotinib (HM781-36B), dacomitinib (PF299804, PF299), AEE788 (NVP-AEE788), AC480 (BMS -599626), TAK-285, lapatinib (GW-572016) ditosylate, and lapatinib, exemplified as the EGF receptor inhibitor, inhibit EGFR, ErbB2 and ErbB4 (Modjtahedi H., et al., 2014, Naunyn-Schmiedeberg's Arch. Pharmacol., 387:505-52; Cha MY et al., 2012, Int. J. Cancer, 130, 2445-2454; Engelman J.A. et al., Cancer Res., 2007;67(24):11924-32; Traxler P. et al., CANCER RESEARCH, 64, 4931-4941, July 15, 2004; Wong T.W. et al., Clin. Cancer Res., 2006;12(20), October 15, 2006; and Doi T. et al., British Journal of Cancer (2012) 106, 666- 672).

Whether the EGF receptor inhibitors mentioned above have an anticancer effect on diffuse gastric cancer can be confirmed by an in vitro cell growth inhibition study, therapeutic efficacy evaluation study in a diffuse gastric cancer model, or the like. In the in vitro cell growth inhibition study, for example, cells of a gastric cancer cell line can be cultured with an EGF receptor inhibitor, and whether growth of the cancer cells is inhibited can be evaluated. Further, cancer cells of a gastric cancer cell line proliferated by culture with a cytokine or growth factor can be further cultured with an EGF receptor inhibitor, and whether growth of the cancer cells is inhibited can be evaluated. As the cytokine and growth factor mentioned above, for example, there can be used those confirmed to be present at a higher concentration in cancer ascites of diffuse gastric cancer patients compared with that in non-cancer ascites. It has been reported that amphiregulin, HB-EGF (heparin binding-epidermal growth factor-like growth factor), HGF (hepatocyte growth factor), etc. are present in the ascites of patients with diffuse gastric cancer at concentrations higher by 2 to 5 times or more compared with those in non-cancer ascites (Non-patent document 2).

The study for evaluating the therapeutic effect in a diffuse gastric cancer model, and the like can be conducted by administering an EGF receptor inhibitor to an animal such as mice grafted with gastric cancer cells, and evaluating the therapeutic effect. The evaluation can be performed by dissecting an euthanized mouse and observing number and size of abdominal tumors, volume of ascites, and the condition of nodules in the peritoneum. The therapeutic effect can be evaluated by administering the EGF receptor inhibitor at the same time as the grafting, or by administering the EGF receptor inhibitor after confirming growth of the tumor for about 5 days. As the model animal, besides mouse, rodents such as rat, rabbit, mini pig, and primates such as crab-eating macaque can be used. In addition to xenograft models using human cancer cells, allogeneic transplantation models, orthotopic transplantation models, chemical carcinogenesis models, and genetically modified animal models can be used. When a cancer cell transplantation model is used, the cancer cell transplantation may be either ectopic or orthotopic transplantation.

Examples of the gastric cancer cell lines include NUGC4, OCUM-1, MKN45, MKN7, MKN28, TMK-1, NKPS, KATOIII, NUGC3, GCIY, H-111-TC, SH -10-TC, KKLS, GCR1, and so forth. Gastric cancer cell lines can be obtained from the Health Science Research Resources Bank (Osaka, Japan), and other sources.

It has been reported that when cells of the gastric cancer cell lines MKN45, NUGC4, GCIY, GCR1, MKN7, and OCUM-1 were cultured with 10 to 200 ng/mL of HGF (hepatocyte growth factor) for 72 hours, twice or more of cell growth was confirmed for the diffuse gastric cancer cell lines NUGC4 and GCIY compared with the baseline growth, but no cell growth was observed for the non-diffuse gastric cancer cell lines MKN45, GCR1, MKN7 and OCUM-1 (Non-patent document 1). It has been reported that HGF is overexpressed in many gastric cancers, and increased serum HGF is associated with poor prognosis (Amemiya et al., Oncology, 2002;63:286-96; and Park et al., APMIS, 2000;108:195-200). In this specification, the baseline growth refers to the growth of cells of cancer cell line cultured in the absence of any cytokine, growth factor, and so forth.

It has been reported that when the diffuse gastric cancer cell line NUGC4 cells were cultured with 100 ng/mL of amphiregulin for 72 hours, about 2 times of cell growth was observed compared with the baseline growth, and when they were cultured with 1 ng/mL of HB-EGF for 72 hours, induction of cell growth was slightly observed (Non-patent document 2). Amphiregulin binds to ErbB1 (EGFR), and HB-EGF binds to both ErbB1 (EGFR) and ErbB4 (Olayioye MAet al., EMBO J., 2000;19:3159-67). If NUGC4 cell line cells are intraperitoneally injected into nude mice, they rapidly disseminate into the abdominal cavities and form bloody ascites (Nakashio T et al., Int. J. Cancer, 1997;70:612-8).

It has been revealed that HGF is highly produced from cancer stromal fibroblasts, which are specifically abundant in diffuse gastric cancer (Non-patent document 1). The cancer stroma-derived HGF induces a potent cell growth effect in addition to increased motility of diffuse gastric cancer cells via the sole receptor thereof, cMet (Non-patent document 1). In addition, this paracrine-inducible HGF induces production of a large amount of amphiregulin, which is one of EGFR ligands having a marked cell growth activity, like HGF (Non-patent document 3), in diffuse gastric cancer cells (Non-patent document 1). Cancer ascites associated with diffuse gastric cancer comprises HGF and amphiregulin at 2 to 5 times higher concentrations compared with those in non-cancer ascites (Non-patent document 2). HGF-induced and self-produced amphiregulin strongly proliferate diffuse gastric cancer cells by the autocrine mechanism.

In contrast, in non-diffuse gastric cancers, there is observed no induction of HGF production from cancer stromal fibroblasts as seen in the cancer stroma of diffuse gastric cancer. In addition, non-diffuse gastric cancer cells do not show HGF- or amphiregulin-stimulated cell growth induction activity, which is a major difference from diffuse gastric cancer cells (Non-patent document 1). It is thought that, in diffuse gastric cancers, activation of the cancer stroma-derived paracrine HGF via the cMet receptor (HGF/cMet axis pathway) plays an important role (Non-patent document 1), and an association thereof with the development of scirrhous gastric cancer and diffuse gastric cancer has been reported also in gastric cancer cells with cMet gene amplification (Nakajima M. et al., Cancer, 1999;85:1894-1902).

The cMet inhibitor showed a high antitumor effect in a mouse carcinomatous peritonitis model using cMet gene-amplified gastric cancer or scirrhous gastric cancer cells (Non-patent document 1). However, although several clinical trials have been conducted with TKI and antibodies targeting the cMet receptor for the whole gastric cancers, sufficient clinical benefit has not been confirmed in any of the trials. Although cMet gene-amplified gastric cancer, which accounts for 2% of total gastric cancers, may be difficult to be classified into diffuse gastric cancer in terms of gross morphology, it has been found to have characteristics of diffuse gastric cancer in terms of molecular mechanisms of growth and development (Nakajima M. et al., Cancer 1999;85; 1894-1902).

The pharmaceutical composition of the present invention can be used for treating diffuse gastric cancer in a patient. According to another embodiment, the pharmaceutical composition of the present invention can be used for treating scirrhous gastric cancer in a patient. If the patient with diffuse gastric cancer mentioned above has carcinomatous peritonitis, it can be used to suppress worsening of the symptoms of carcinomatous peritonitis or to relieve or cure the symptoms. The pharmaceutical composition of the present invention can also be used for preventing development of carcinomatous peritonitis in the case where the patient with diffuse gastric cancer mentioned above has not developed carcinomatous peritonitis. In this specification, carcinomatous peritonitis refers to a pathological condition in which cancer has metastasized to the peritoneum, and it is caused by dissemination of cancer cells into the abdominal cavity, which results in formation of numerous nodules of cancer cells on the peritoneum (peritoneal dissemination). Carcinomatous peritonitis is often accompanied by malignant ascites as peritoneal dissemination increases. Carcinomatous peritonitis is also referred to as peritoneal carcinomatosis. The pharmaceutical composition of the present invention can be used for treating gastric cancer patients suffering from carcinomatous peritonitis.

Furthermore, the aforementioned EGF receptor inhibitors can have an inhibitory activity on the growth of fibroblasts in gastric tissues. It is known that the interaction between tumor cells and stromal cells plays an important role in tumor development and progression, and stromal fibroblasts are often associated with cancer progression (Kalluri R et al., Nat. Rev. Cancer, 2006;6:392-401). Scirrhous gastric cancer is characterized by rapid diffuse invasion and growth of cancer cells as well as high degree of stromal fibrosis. Non-patent document 2 reports that in scirrhous gastric cancer, normal human fibroblasts existing in the tumor microenvironment of the stomach and abdominal cavity express the EGFR protein at a high level, and that HB-EGF induces migration and growth of fibroblasts existing in the stroma. Therefore, HB-EGF produced in the peritoneal cavity may promote accumulation and growth of fibroblasts, and may induce fibrosis associated with peritoneal carcinomatosis in patients with scirrhous gastric cancer.

The pharmaceutical composition of the present invention may be administered according to known clinical practice. When the pharmaceutical composition of the present invention is orally administered, it may be administered so that the dose of the EGF receptor inhibitor is 100 µg to 10 g, 500 µg to 10 g, or 1 mg to 1 g, per day for an adult (body weight 60 kg). When the EGF receptor inhibitor used in the present invention is osimertinib (AZD9291), the dose thereof is, for example, 80 mg per day. If the EGF receptor inhibitor used in the present invention is AST1306, the dose thereof is, for example, 1000 to 1200 mg per day. The EGF receptor inhibitor used in the present invention can be administered one to three times per day as divided doses.

The pharmaceutical composition provided by the present invention is formulated in an optimal dosage form depending on the route of administration. The pharmaceutical composition may be in a dosage form for oral administration including a solid preparation such as tablet, granule, fine granule, powder, and capsule, liquid, jelly, syrup, and so forth, or a dosage form for parenteral administration such as injection, suppository, ointment, and patch.

The pharmaceutical composition provided by the present invention may contain pharmaceutically acceptable additives. Examples of pharmaceutically acceptable additives include, for example, isotonic agent, pH adjuster, buffer, stabilizer, cryoprotectant, antibiotic, and so forth. Specific examples include water, ethanol, sodium chloride, glucose, albumin, and so forth. Other examples of the pharmaceutically acceptable additives include excipient, binder, lubricant, and so forth. Examples of excipient include, for example, lactose, cornstarch, sucrose, glucose, sorbitol, crystalline cellulose, silicon dioxide, and so forth. Examples of binder include, for example, polyvinyl alcohol, ethylcellulose, methylcellulose, gum arabic, hydroxypropylcellulose, hydroxypropylmethylcellulose, and so forth. Examples of lubricant include, for example, magnesium stearate, talc, silica, and so forth.

The pharmaceutical composition of the present invention should be preferably manufactured under conditions that conform to the regulations for manufacturing and quality control of pharmaceuticals and quasi-pharmaceuticals (Good Manufacturing Practice (GMP)).

The pharmaceutical composition provided by the present invention can be used in a combination therapy with an anti-VEGF receptor 2 antibody and/or a cMET inhibitor. According to one embodiment, the pharmaceutical composition of the present invention can be used in a combination therapy with an anti-VEGF receptor 2 antibody. According to another embodiment, the pharmaceutical composition of the present invention can be used in a combination therapy with a cMET inhibitor. In yet another embodiment, the pharmaceutical composition of the present invention can be used in a combination therapy with an anti-VEGF receptor 2 antibody and a cMET inhibitor.

The VEGF receptor 2 (also referred to as VEGFR2 in this specification) refers to the vascular epidermal growth factor receptor (VEGFR) 2. The VEGF receptor 2 is also known as KDR. As the anti-VEGF receptor 2 antibody, any antibody that binds to the VEGF receptor 2 can be used. According to one embodiment, the anti-VEGF receptor 2 antibody is ramucirumab. Ramucirumab is marketed as Cyramza (registered trademark), and also known as IMC-1121b or CAS Registry Number 947687-13-0. The anti-VEGF receptor 2 antibody DC101 is a rat monoclonal antibody directed to the mouse VEGF receptor 2, which can be used in experiments as a substitute for anti-VEGF receptor 2 antibodies, preferably ramucirumab, in mice (Witte L., et al., Cancer Metastasis Rev., 155-161, 1998).

Examples of the aforementioned cMET inhibitor include crizotinib (PF-02341066), TAS-115, cabozantinib (XL184, BMS-907351), foretinib, PHA-665752, SU11274, SGX-523, BMS-777607, JNJ-38877605, tivantinib (ARQ197), PF-04217903, glesatinib (MGCD265), capmatinib (INCB28060), BMS-754807, BMS-794833, AMG-208, MK-2461, golvatinib (E7050), AMG-458, NVP-BVU972, norcantharidin, AMG337, HMPL-504 (AZD6094, savolitinib), S49076, NPS-1034, and merestinib (LY2801653). The inhibitor may be a pharmaceutically acceptable salt of these mentioned above. According to one embodiment of the present invention, the cMET inhibitor is savolitinib.

In the combination therapy, two or more kinds of drugs may be administered simultaneously or sequentially. When two or more kinds of drugs are administered simultaneously or sequentially, all of the drugs may be administered through the same route or different routes.

In this specification, simultaneous administration means that two or more kinds of drugs are administered with a time interval not longer than several minutes to several seconds. For example, two kinds of drugs are administered with a time interval not longer than about 15 minutes to 1 minute. In this specification, sequential administration means that the drugs are administered with a time interval of several minutes, several hours, several days, or several weeks. For example, two kinds of drugs are administered with a time interval of 15 minutes or longer, 30 minutes or longer, 60 minutes or longer, or 1 day, 2 days, 3 days, 4 days, 5 days, 6 days or 7 days, or 2 weeks, 3 weeks or 4 weeks.

In the combination therapy, the EGF receptor inhibitor may be administered simultaneously or sequentially with the anti-VEGF receptor 2 antibody. In the combination therapy, the EGF receptor inhibitor may be administered simultaneously or sequentially with the cMET inhibitor. In the combination therapy, the EGF receptor inhibitor can be administered simultaneously or sequentially with the anti-VEGF receptor 2 antibody and cMET inhibitor.

According to one embodiment, the EGF receptor inhibitor contained in the pharmaceutical composition of the present invention is AZ5104, and it can be used in a combination therapy with savolitinib. According to another embodiment, the EGF receptor inhibitor contained in the pharmaceutical composition of the present invention is osimertinib, and it can be used in a combination therapy with savolitinib.

According to one embodiment, the EGF receptor inhibitor contained in the pharmaceutical composition of the present invention is AZ5104, and it can be used in a combination therapy with ramucirumab. According to another embodiment, the EGF receptor inhibitor contained in the pharmaceutical composition of the present invention is osimertinib, and it can be used in a combination therapy with ramucirumab.

According to one embodiment, the EGF receptor inhibitor contained in the pharmaceutical composition of the present invention is AZ5104, and it can be used in a combination therapy with savolitinib and ramucirumab. According to another embodiment, the EGF receptor inhibitor contained in the pharmaceutical composition of the present invention is osimertinib, and it can be used in a combination therapy with savolitinib and ramucirumab.

The EGF receptor inhibitor may be administered at a dose of 100 µg to 10 g, 500 µg to 10 g, or 1 mg to 1 g, per day for an adult (body weight 60 kg) in combination use with the anti-VEGF receptor 2 antibody and/or cMET inhibitor. When the EGF receptor inhibitor used in the present invention is osimertinib (AZD9291), an example of the dose is 80 mg per day. If the EGF receptor inhibitor used in the present invention is AST1306, an example of the dose is 1000 to 1200 mg per day. The EGF receptor inhibitor used in the present invention can be administered one to three times per day as divided doses.

In combination use with the EGF receptor inhibitor and/or the cMET inhibitor, ramucirumab can be administered to an adult at a single dose of 8 mg/kg (body weight) to 10 mg/kg (body weight) in terms of ramucirumab once every two to three weeks, but the dose can be appropriately reduced depending on the patient's condition. For example, it can be administered by intravenous drip infusion at a single dose of 8 mg/kg (body weight) to 10 mg/kg (body weight) over approximately 60 minutes.

In combination use with the EGF receptor inhibitor and/or anti-VEGF receptor 2 antibody, savolitinib can be orally administered to an adult at a dose of 300 mg to 1200 mg, preferably 600 mg, per day, and can be administered one to three times per day as divided doses.

### (Treatment method)

The present invention provides a method for treating diffuse gastric cancer in a patient, which comprises the step of administering an EGF receptor inhibitor having an ErbB1 inhibitory activity and an ErbB4 inhibitory activity to the patient. In the treatment method using the pharmaceutical composition of the present invention, the EGF receptor inhibitor can be orally, intragastrically, intranasally, intralesionally, or topically administered by any suitable means. The pharmaceutical composition of the present invention may be administered by parenteral injection, which includes intramuscular, intravenous, intraarterial, or intraperitoneal injections, and subcutaneous injection. The administration regimen is appropriately planned depending on whether the administration is short or long-term administration. A therapeutically optimal and clinically appropriate administration route, administration method, dose, and administration schedule are decided on the basis of knowledge of medical practitioners.

The treatment method of the present invention can be performed on patients whose expression levels or level of ErbB1 and/or ErbB4 proteins in a patient-derived gastric cancer cell sample is statistically equivalent to or higher than a predetermined control level. The control level may be an average of expression levels of ErbB1 and/or ErbB4 protein in multiple specimens of diffuse gastric cancers that are sensitive to the EGF receptor inhibitor.

The treatment method of the present invention can be performed on patients whose ErbB1 and/or ErbB4 protein in patient-derived gastric cancer cell samples is phosphorylated or unphosphorylated. According to one embodiment, the treatment method of the present invention can be selectively performed on patients whose ErbB1 and/or ErbB4 protein in a patient-derived gastric cancer cell sample is phosphorylated. The aforementioned EGF receptor inhibitor may have an inhibitory activity against phosphorylated ErbB1 and/or ErbB4 protein.

As the treatment method of the present invention, an EGF receptor inhibitor may be applied to a patient as a single therapy, or it may be accompanied by conventional surgery, radiation therapy, chemotherapy, administration of antibody drug, or immunotherapy in addition to administration of the EGF receptor inhibitor. A drug for such chemotherapy and antibody drug may be administered simultaneously, sequentially, or separately, along with performing the EGF receptor inhibitor-based therapy.

In the treatment method of the present invention, the drug used for the chemotherapy performed in combination with administration of the EGF receptor inhibitor may be selected from such anticancer agents as oxaliplatin, capecitabine, fluorouracil, irinotecan, cisplatin, docetaxel, paclitaxel, tegafur, doxifluridine, tegafur-uracil mixture, tegafur-gimeracil-oteracil potassium preparation, and so forth.

In the treatment method of the present invention, the antibody drug used in combination with the EGF receptor inhibitor can be selected from such anticancer drugs as, but not limited to, the anti-VEGF receptor 2 antibody ramucirumab, anti-PD-1 antibody nivolumab, anti-PD-1 antibody pembrolizumab, anti-PD-L1 antibody avelumab, and anti-HER2 antibody, veltuzumab.

In the treatment method of the present invention, the step of administering an EGF receptor inhibitor having an ErbB1 inhibitory activity and an ErbB4 inhibitory activity to a patient may comprise simultaneously or sequentially administering the EGF receptor inhibitor with an anti-VEGF receptor 2 antibody. According to one embodiment, the anti-VEGF receptor 2 antibody is ramucirumab.

In the treatment method of the present invention, the step of administering an EGF receptor inhibitor having an ErbB1 inhibitory activity and an ErbB4 inhibitory activity to a patient may comprise simultaneously or sequentially administering the EGF receptor inhibitor with a cMET inhibitor. In other words, in the method of the present invention for treating diffuse gastric cancer in a patient, the EGF receptor inhibitor can be administered in combination with a cMET inhibitor. cMET is a tyrosine kinase type receptor, and it is activated by binding of HGF (hepatocyte growth factor). The activation of the HGF/cMET signaling pathway induces cell growth, migration, morphogenesis, angiogenesis, and so forth, and its sustained activation is thought to be involved in tumor growth. The aforementioned cMET inhibitor generally has an activity to inhibit the HGF-cMet signaling pathway. Any appropriate cMET receptor inhibitor that has a tyrosine kinase inhibitory activity and/or inhibits signaling from the cMET receptor may be used.

Examples of the aforementioned cMET inhibitor include crizotinib (PF-02341066), TAS-115, cabozantinib (XL184, BMS-907351), foretinib, PHA-665752, SU11274, SGX-523, BMS-777607, JNJ-38877605, tivantinib (ARQ197), PF-04217903, glesatinib (MGCD265), capmatinib (INCB28060), BMS-754807, BMS-794833, AMG-208, MK-2461, golvatinib (E7050), AMG -458, NVP-BVU972, norcantharidin, AMG337, HMPL-504 (AZD6094, savolitinib), S49076, NPS-1034, and merestinib (LY2801653). The inhibitors may be a pharmaceutically acceptable salt of these exemplified above. According to one embodiment of the present invention, the cMET inhibitor is savolitinib.

Crizotinib (PF-02341066) is 3-((R)-1-(2,6-dichloro-3-fluorophenyl)ethoxy)-5-(1-(piperidin-4-yl)-1H-pyrazol-4-yl)pyridin-2-amine (CAS Registry Number: 877399-52- 5). Cabozantinib (XL184, BMS-907351) is N-(4-(6,7-dimethoxyquinolin-4-yloxy)phenyl)-N-(4-fluorophenyl)cyclopropane-1,1-dicarboxamide (CAS Registry Number: 849217-68-1). Foretinib is N-(3-fluoro-4-((6-methoxy-7-(3-morpholinopropoxy)quinolin-4-yl)-oxy)phenyl)-N-(4-fluorophenyl)cyclopropane-1,1-dicarboxamide (CAS Registry Number: 849217-64-7). CAS Registry Number of TAS-115 is 1190836-34-0. PHA-665752 is (R,Z)-5-(2,6-dichlorobenzylsulfonyl)-3-((3,5-dimethyl-4-(2-(pyrrolidin-1-ylmethyl)pyrrolidin-1-carbonyl)-1H-pyrrol-2 -yl)methylene)indolin-2-one (CAS Registry Number: 477575-56-7). SU11274 is (Z)-N-(3-chlorophenyl)-3-((3,5-dimethyl-4-(1-methylpiperazine-4-carbonyl)-1H-pyrrol-2-yl)methylene-N-methyl-2-oxoindoline-5-sulfonamide (CAS Registry Number: 658084-23-2). SGX-523 is 6-(6-(1-methyl-1H-pyrazol-4-yl)-[1,2,4]triazolo[4,3-b]pyridazin-3-ylthio)quinoline (CAS Registry Number: 1022150-57-7).

BMS-777607 is N-(4-(2-amino-3-chloropyridin-4-yloxy)-3-fluorophenyl)-4-ethoxy-1-(4-fluorophenyl)-2-oxo-1,2-dihydropyridine-3-carboxamide (CAS Registry Number: 1025720-94-8). JNJ-38877605 is 6-(difluoro(6-(1-methyl-1H-pyrazol-4-yl)-[1,2,4]triazolo[4,3-b]pyridazin-3-yl)methyl)quinoline (CAS Registry Number: 943540-75-8). Tivantinib (ARQ197) is (3R,4R)-3-(2,3-dihydro-1H-pyrrolo[3,2,1-ij]quinolin-6-yl)-4-(1H-indol-3-yl)pyrrolidine-2,5-dione (CAS Registry Number: 905854-02-6). PF-04217903 is 2-(4-(3-(quinolin-6-ylmethyl)-3H-[1,2,3]triazolo[4,5-b]pyrazin-5-yl)-1H-pyrazol-1-yl)ethanol (CAS Registry Number: 956905-27-4). CAS Registry Number of glesatinib (MGCD265) is 936694-12-1. Capmatinib (INCB28060) is 2-fluoro-N-methyl-4-(7-(quinolin-6-ylmethyl)imidazo[1,2-b][1,2,4]triazin-2-yl)benzamide (CAS Registry Number: 1029712-80-8). BMS-754807 is (S)-1-(4-(5-cyclopropyl-1H-pyrazol-3-ylamino)pyrrolo[1,2-f][1,2,4]triazin-2-yl)-N-(6-fluoropyridin-3-yl)-2-methylpyrrolidine-2-carboxamide (CAS Registry Number: 1001350-96-4). BMS-794833 is N-(4-(2-amino-3-chloropyridin-4-yloxy)-3-fluorophenyl)-5-(4-fluorophenyl)-4-oxo-1,4-dihydropyridine-3-carboxamide (CAS Registry Number: 1174046-72-0).

AMG-208 is 7-methoxy-4-((6-phenyl-[1,2,4]triazolo[4,3-b]pyridazin-3-yl)methoxy)quinoline (CAS Registry Number: 1002304-34-8). MK-2461 is N-((2R)-1,4-Dioxan-2-ylmethyl)-N-methyl-N'-[3-(1-methyl-1H-pyrazol-4-yl)-5-oxo-5H-benzo[4,5]cyclohepta[1,2-b]pyridin-7-yl]sulfamide (CAS Registry Number: 917879-39-1). Golvatinib (E7050) is 1,1-Cyclopropanedicarboxamide, N-[2-fluoro-4-[[2-[[[4-(4-methyl-1-piperazinyl)-1-piperidinyl]carbonyl]amino]-4-pyridinyl]oxy]phenyl]-N'-(4-fluorophenyl)- (CAS Registry Number: 928037-13-2). AMG-458 is 1-(2-hydroxy-2-methylpropyl)-N-(5-(7-methoxyquinolin-4-yloxy)pyridin-2-yl)-5-methyl-3-oxo-2-phenyl-2,3-dihydro-1H-pyrazole-4-carboxamide (CAS Registration Number: 913376-83-7). NVP-BVU972 is 6-((6-(1-methyl-1H-pyrazol-4-yl)imidazo[1,2-b]pyridazin-3-yl)methyl)quinoline (CAS Registration Number: 1185763-69-2). Norcantharidin is exo-3,6-epoxyhexahydrophthalic anhydride (CAS Registry Number: 29745-04-8).

AMG337 is 1,6-Naphthyridin-5(6H)-one, 6-[(1R)-1-[8-fluoro-6-(1-methyl-1H-pyrazol-4-yl)-1,2,4-triazolo[4,3-a]pyridin-3-yl]ethyl]-3-(2-methoxyethoxy)- (CAS Registry Number: 1173699-31-4). HMPL-504 (AZD6094, savolitinib) is 1H-1,2,3-Triazolo[4,5-b]pyrazine, 1-[(1S)-1-imidazo[1,2-a]pyridin-6-yl]ethyl]-6-(1-methyl-1H-pyrazol-4-yl)- (CAS Registry Number; 1313725-88-0). S49076 is 2,4-thiazolidinedione, 3-[[2,3-dihydro-3-[[4-(4-morpholinylmethyl)-1H-pyrrol-2-yl]methylene]-2-oxo-1H-indol-5-yl]methyl]- (CAS Registry Number: 1265965-22-7). NPS-1034 is N-(3-fluoro-4-(3-phenyl-1H-pyrrolo[2,3-b]pyridin-4-yloxy)phenyl)-2-(4-fluorophenyl)-1,5-dimethyl-3-oxo-2,3-dihydro-1H-pyrazole-4-carboxamide (CAS Registry Number 122713-92-3). Merestinib (LY2801653) is a 3-pyridinecarboxamide, N-[3-fluoro-4-[[1-methyl-6-(1H-pyrazol-4-yl)-1H-indazol-5-yl]oxy]phenyl]-1-(4-fluorophenyl)-1,2-dihydro-6-methyl-2-oxo- (CAS Registry Number: 1206799-15-6).

The examples of the present invention described below are mentioned only for illustrative purposes, and are not intended to limit the technical scope of the present invention. The technical scope of the present invention is limited only by the descriptions of the claims. The present invention may be modified by, for example, adding, deleting, and substituting components of the invention, unless such modifications depart from the scope of the present invention.

### Examples

### (Example 1)

### In vitro cell growth inhibition experiments against gastric cancer cell lines

The effects of the EGFR inhibitors, AST1306 and AZ5104, on the growth of gastric cancer cell lines were investigated.

The following cell lines were used for the in vitro cell growth inhibition experiments.
(1) Diffuse gastric cancer cell lines
   - NUGC4 (obtained from the Health Science Research Resources Bank, Osaka, Japan)
   - GCIY (obtained from the RIKEN BioResource Center, Tsukuba, Japan)
(2) Highly differentiated non-diffusive human gastric cancer cell lines
   - MKN7 (obtained from the Health Science Research Resources Bank, Osaka, Japan)
   - GCR1 (obtained from the Cancer Research Institute, Kanazawa University)
(3) cMet gene-amplified gastric cancer cell line
   - MKN45 (obtained from the Health Science Research Resources Bank, Osaka, Japan)

The EGFR inhibitors used were as follows.
- AST1306 (obtained from Selleck Chemicals)
- AZ5104 (obtained from Selleck Chemicals)

Five types of cell lines (NUGC4, GCIY, MKN7, GCR1 and MKN45) were subcultured in the RPMI 1640 culture medium containing 10% concentration of fetal bovine serum, 100 units/mL concentration of penicillin, and 100 units/mL concentration of antibiotic streptomycin, and used in the following experiments. The cells were periodically examined for the presence of mycoplasma infection by using MycoAlert Mycoplasma Detection Kit (Lonza).

Cell growth was measured by using Cell Counting Kit-8 (CCK-8) (Dojin Kagaku Kenkyusho, Kumamoto, Japan) (Ishiyama M., et al., Talenta, 44;1299:1997). The cells were inoculated at a density of 5 x 10⁴ cells/mL in a volume of 100 µL per well, and incubated at 37°C for 24 hours in an incubator. Then, any one of three types of cell growth factors, amphiregulin (AP, 100 ng/mL, R&D Systems, Minneapolis, MN), heparin-binding EGF-like growth factor (HB-EGF), and hepatocyte growth factor (HGF, 50 ng/mL, R&D Systems), and any one of two types of the EGFR inhibitors, AZ5104 (0.001 to 10 µM) and AST1306 (0.001 to 10 µM)), were added to each well, and the cells were further incubated at 37°C for 72 hours in an incubator. In addition, to the control wells, any one of the three types of cell growth factors was added, but not the EGFR inhibitor. Further, 10 µL of a WST-8 solution was added to each well, and the cells were cultured at 37°C for 4 hours. Absorbance was measured at a wavelength of 450 nm by using a microplate reader (BIO-RAD Laboratories, Inc.). All the experiments were performed in triplicate, that is, the same experiment was repeated three times.

The results are shown in Figs. 1 to 3.

### (Diffuse gastric cancer cell lines)

The results of the cell growth inhibition test for the diffuse gastric cancer cell lines NUGC4 and GCIY are shown in Fig. 1.

For the diffuse gastric cancer cell line NUGC4, amphiregulin-induced cell growth, HB-EGF-induced cell growth, and HGF-induced cell growth were observed. For the diffuse gastric cancer cell line GCIY, amphiregulin-induced cell growth, HB-EGF-induced cell growth, and HGF-induced cell growth were observed, but the amplification rates were lower than those observed for NUGC4.

### (1) AST1306

AST1306 inhibited the amphiregulin-induced cell growth, HB-EGF-induced cell growth, and HGF-induced cell growth of the diffuse gastric cancer cell line NUGC4 in a dose-dependent manner.

AST1306 inhibited the amphiregulin-induced cell growth, HB-EGF-induced cell growth, and HGF-induced cell growth of the diffuse gastric cancer cell line GCIY in a dose-dependent manner. In addition, AST1306 inhibited the baseline cell growth of the diffuse gastric cancer cell line GCIY.

### (2) AZ5104

AZ5104 inhibited the amphiregulin-induced cell growth, HB-EGF-induced cell growth, and HGF-induced cell growth of the diffuse gastric cancer cell line NUGC4 in a dose-dependent manner.

AZ5104 inhibited the amphiregulin-induced cell growth, HB-EGF-induced cell growth, and HGF-induced cell growth of the diffuse gastric cancer cell line GCIY in a dose-dependent manner. In addition, AZ5104 inhibited the baseline cell growth of the diffuse gastric cancer cell line GCIY.

### (Non-diffuse gastric cancer cell lines)

The results of cell growth inhibition tests for the non-diffuse gastric cancer cell lines GCR-1 and MKN7 are shown in Fig. 2.

For the non-diffuse gastric cancer cell lines GCR-1 and MKN7, amphiregulin-induced cell growth and HB-EGF-induced cell growth were hardly observed, and HGF-induced cell growth was slightly observed.

### (1) AST1306

AST1306 inhibited cell growth of the non-diffuse gastric cancer cell lines GCR-1 and MKN7 at a concentration of 0.1 or 0.3 µM or higher. AST1306 also inhibited the HGF-induced cell growth of non-diffuse gastric cancer cell lines GCR-1 and MKN7 at a concentrations of 0.3 µM or higher.

### (2) AZ5104

AZ5104 inhibited cell growth of the non-diffuse gastric cancer cell lines GCR-1 and MKN7 at a concentration of 0.1 or 0.3 µM or higher. AZ5104 also inhibited the HGF-induced cell growth of the non-diffuse gastric cancer cell lines GCR-1 and MKN7 at a concentration of 0.3 µM or higher.

### (cMet gene-amplified gastric cancer cell line)

The results of the cell growth inhibition test for the cMet gene-amplified gastric cancer cell line MKN45 are shown in Fig. 3.

For the cMet gene-amplified gastric cancer cell line MKN45, amphiregulin-induced cell growth, HB-EGF-induced cell growth, and HGF-induced cell growth were not substantially observed.

### (1) AST1306

AST1306 inhibited cell growth of the cMet gene-amplified gastric cancer cell line, MKN45, at a concentrations of 1 µM or higher.

### (2) AZ5104

AZ5104 inhibited cell growth of the cMet gene-amplified gastric cancer cell line, MKN45, at a concentrations of 1 µM or higher.

### (Example 2)

### In vitro growth inhibition test for fibroblasts

The effects of AZ5104 and AST1306 on the growth of human fibroblasts were examined.

Normal human gastric fibroblasts established at the Department of Medical Oncology, Kanazawa Medical University were subcultured in the RPMI 1640 culture medium (containing 10% concentration of fetal bovine serum) containing two kinds of antibiotics, penicillin (Life Technologies, Carlsbad, CA, USA) and streptomycin (Life Technologies, Carlsbad, CA, USA), at a concentration of 100 U/mL, respectively, and used for the experiments. The cells were periodically tested for the presence of mycoplasma infection by using the MycoAlert Mycoplasma Detection Kit (Lonza).

Using a 6-well plate for cell culture, 2 mL of the RPMI 1640 culture medium (1% concentration of fetal bovine serum) containing 5.5 x 10⁴ of the human fibroblasts and two kinds of the antibiotics mentioned above was inoculated to each well, and the cells were cultured overnight at 37°C in a CO₂ incubator. Then, 1 µg/mL concentration of human normal IgG or anti-human HB-EGF antibody (R&D systems, Minneapolis, MN) was added to each well, and the cells were further incubated at 37°C for 24 hours in a CO₂ incubator. Thereafter, there were added human HB-EGF (R&D Systems, Minneapolis, MN) at a concentration of 10 ng/mL, and the EGF receptor inhibitor AZ5104 or AST1306 at a concentration of 1 µM to each well, and the cells were further incubated at 37°C for 96 hours in a CO₂ incubator. After the above treatment, the cells were treated with a 0.25% trypsin/EDTA solution (Life Technologies, Carlsbad, CA, USA) and counted. The same experiment was repeated three times.

The results are shown in Fig. 4.

Cell growth of the normal human gastric fibroblasts was induced by HB-EGF, and the HB-EGF-induced cell growth was inhibited by the administration of any of AST1306, AZ5104, and anti-human HB-EGF antibody. Administration of human normal IgG, used as a control, did not inhibit the HB-EGF-induced cell growth.

### (Example 3)

### In vivo test

AZ5104, osimertinib (AZD9291), and savolitinib (HMPL-504, AZD6094) were obtained from Selleck Chemicals. The anti-VEGF receptor 2 antibody used was antimouse VEGF receptor 2 antibody DC101 (GeneTex).

Diffuse gastric cancer cells (NUGC4 cells) were transplanted to Balb/c nu/nu mice (6 weeks old) by intraperitoneal administration of 2 x 10⁶ of the cells in 200 µL of phosphate buffered saline (PBS) per mouse. On day 21 after the transplantation, ascites accumulation was confirmed, and treatment was started. The treatment was performed with the following eight kinds of therapies, and eight mice were used for each therapy.
(1) AZ5104 monotherapy: Administration of AZ5104 once a day at a single dose of 10 mg/kg (body weight)
(2) Osimertinib monotherapy: Administration of osimertinib once a day at a single dose of 10 mg/kg (body weight)
(3) Savolitinib monotherapy: Administration of savolitinib once a day at a single dose of 2.5 mg/kg (body weight)
(4) Anti-VEGF receptor 2 antibody monotherapy: Administration of anti-VEGF receptor 2 antibody twice a week at a single dose of 40 mg/kg (body weight)
(5) AZ5104 and savolitinib combination therapy: Combination of administration of AZ5104 once a day at a single dose of 10 mg/kg (body weight) and administration of savolitinib once a day at a single dose of 2.5 mg/kg (body weight)
(6) Osimertinib and savolitinib combination therapy: Combination of administration of osimertinib once a day at a single dose of 10 mg/kg (body weight) and administration of savolitinib once a day at a single dose of 2.5 mg/kg (body weight)
(7) AZ5104, savolitinib, and anti-VEGF receptor 2 antibody combination therapy: Combination of administration of AZ5104 once a day at a single dose of 10 mg/kg (body weight), administration of savolitinib once a day at a single dose of 2.5 mg/kg (body weight), and administration of anti-VEGF receptor 2 antibody twice a week at a single dose of 40 mg/kg (body weight)
(8) Osimertinib, savolitinib, and anti-VEGF receptor 2 antibody combination therapy: Combination of administration of osimertinib once a day at a single dose of 10 mg/kg (body weight), administration of savolitinib once a day at a single dose of 2.5 mg/kg (body weight), and administration of anti-VEGF receptor 2 antibody twice a week at a single dose of 40 mg/kg (body weight).

AZ5104, osimertinib, and savolitinib were orally administered, and the anti-VEGF receptor 2 antibody was intraperitoneally administered.

For the control group (vehicle control), only a solution not dissolving any drug was administered once a week. The administration was continued for 4 weeks, and then for another 4 weeks.

### Results:

All the mice of the control group showed ascites formation and died within 5 weeks after the gastric cancer cell transplantation.

The mice that were alive 11 weeks after the gastric cancer cell transplantation were 3 mice in the osimertinib and savolitinib combination therapy group, 2 mice in the AZ5104, savolitinib and anti-VEGF receptor 2 antibody combination therapy group, 2 mice in the osimertinib, savolitinib and anti-VEGF receptor 2 antibody combination therapy group, 2 mice in the osimertinib monotherapy group, and 1 mouse in the AZ5104 and savolitinib combination therapy group.

Seven weeks after the gastric cancer cell transplantation, 7 mice in the AZ5104, savolitinib and anti-VEGF receptor 2 antibody combination therapy group, and 8 mice in the osimertinib, savolitinib and anti-VEGF receptor 2 antibody combination therapy group were alive, while three or fewer mice were alive in the monotherapy and two-drug combination therapy groups.

Seven weeks after the gastric cancer cell transplantation, one mouse in the AZ5104 monotherapy group, 2 mice in the savolitinib monotherapy group, and 3 mice in the anti-VEGF receptor 2 antibody monotherapy group were alive, but all died before 11 weeks passed after the gastric cancer cell transplantation.

The t-test performed for the numbers of mice alive 11 weeks after the gastric cancer cell transplantation revealed that there was no significant difference in the number of mice for the savolitinib monotherapy group compared with the control group, but there were significant differences for the other treatment groups, i.e., AZ5104 monotherapy group, osimertinib monotherapy group, anti-VEGF receptor 2 antibody monotherapy group, the two-drug combination therapy groups, and the three-drug combination therapy groups (p<0.05).

Fig. 5 shows the survival curves. The survival curves were created by the Kaplan-Meier method, and the difference of the survival curves between two groups was tested by the log-rank test. The log-rank test performed for the survival rate against the control group revealed that the anti-VEGF receptor 2 antibody monotherapy group, the AZ5104 and savolitinib combination therapy group, the AZ5104, savolitinib and anti-VEGF receptor 2 antibody combination therapy group, and the osimertinib, savolitinib and anti-VEGF receptor 2 antibody combination therapy group showed significant differences (P<0.05). The log-rank test performed for each of the monotherapy groups revealed that the three-drug combination therapy groups (AZ5104, savolitinib and anti-VEGF receptor 2 antibody combination therapy group, and osimertinib, savolitinib, and anti-VEGF receptor 2 antibody combination therapy group) showed significant differences against each of the AZ5104 monotherapy group, the savolitinib monotherapy group, and the anti-VEGF receptor 2 antibody monotherapy group (P<0.05). In particular, significant differences in improvement of the survival rate were observed for the AZ5104, savolitinib, and anti-VEGF receptor 2 antibody combination therapy group, and the osimertinib, savolitinib, and anti-VEGF receptor 2 antibody combination therapy group.

## Claims

1. A pharmaceutical composition for treating diffuse gastric cancer in a patient, which comprises an EGF receptor inhibitor having an ErbB1 inhibitory activity and an ErbB4 inhibitory activity.

2. The pharmaceutical composition according to claim 1, which is for use in a combination therapy with an anti-VEGF receptor 2 antibody and/or a cMET inhibitor.

3. The pharmaceutical composition according to claim 2, wherein the EGF receptor inhibitor is simultaneously or sequentially administered with the anti-VEGF receptor 2 antibody and/or the cMET inhibitor in the combination therapy.

4. The pharmaceutical composition according to any one of claims 1 to 3, wherein the EGF receptor inhibitor is any one selected from the group consisting of afatinib (BIBW2992), AZ5104, osimertinib (AZD9291), poziotinib (HM781-36B), dacomitinib (PF299804, PF299), AEE788 (NVP-AEE788), AC480 (BMS-599626), TAK-285, lapatinib (GW-572016) ditosylate, and lapatinib, or a pharmaceutically acceptable salt thereof.

5. The pharmaceutical composition according to any one of claims 1 to 4, wherein the EGF receptor inhibitor is any one selected from the group consisting of AZ5104 and osimertinib (AZD9291), or a pharmaceutically acceptable salt thereof.

6. The pharmaceutical composition according to any one of claims 2 to 5, wherein the anti-VEGF receptor 2 antibody is ramucirumab.

7. The pharmaceutical composition according to any one of claims 2 to 6, wherein the cMET inhibitor is savolitinib.

8. The pharmaceutical composition according to any one of claims 1 to 7, wherein the diffuse gastric cancer is scirrhous gastric cancer.

9. The pharmaceutical composition according to any one of claims 1 to 8, wherein the patient suffers from carcinomatous peritonitis.

10. A method for treating diffuse gastric cancer in a patient, which comprises the step of administering an EGF receptor inhibitor having an ErbB1 inhibitory activity and an ErbB4 inhibitory activity to the patient.

11. The treatment method according to claim 10, wherein the administration step comprises administering the EGF receptor inhibitor simultaneously or sequentially with an anti-VEGF receptor 2 antibody.

12. The treatment method according to claim 11, wherein the administration step comprises administering the EGF receptor inhibitor and the anti-VEGF receptor 2 antibody simultaneously or sequentially with a cMET inhibitor.
